# EUROPEAN PATENT APPLICATION

(11) **EP 4 706 503 A2**
(43) Date of publication of application: **11.03.2026**
(21) Application number: 26152391.4
(22) Date of filing: 25.03.2024
(51) Int. Cl.: A61B 3/15

(54) **OPHTHALMIC RANGING INSTRUMENT**

(62) Divisional of application: 24165816.0
(71) Applicant: Optos PLC, Dunfermline, Scotland KY11 8GR (GB)
(72) Inventor: PINNOCK, Ralph, Dunfermline, Scotland, KY11 8GR (GB); BUSTIN, Nicholas, Dunfermline, Scotland, KY11 8GR (GB)
(74) Representative: Hoffmann Eitle

(57) **Abstract**

A system comprising an ophthalmic apparatus arranged to determine a distance between the ophthalmic apparatus and an eye, the ophthalmic apparatus comprising: an illumination device which illuminates the eye; an imaging device which acquires an image of the eye, the illumination device and the imaging device being arranged such that the image of the eye comprises a specular highlight from the illumination device having a spatial distribution in the image which varies with the distance; and a processor arranged to process the image to determine an indication of a property of the spatial distribution, and use the indication to determine the distance between the ophthalmic apparatus and the eye. The system further comprising a movement mechanism to move the ophthalmic apparatus towards and away from the eye. The processor determines whether the determined distance is within a predefined range. Where the determined distance is outside the predefined range, the processor generates a control signal for adjustment of the distance by the movement mechanism towards the predefined range. Where the determined distance is within the predefined range, the processor is further arranged to: process acquired stereo images of the eye using a stereoscopic ranging technique to determine a second indication of the distance between the ophthalmic apparatus and the eye; determine whether the distance indicated by the determined second indication is within a second predefined range; and where the distance indicated by the second indication is outside the second predefined range, generate a second control signal for adjustment of the distance between the ophthalmic apparatus and the eye by the movement mechanism towards the second predefined range.

## Description

### [Field]

Example aspects herein generally relate to the field of ophthalmic devices and, in particular, to ophthalmic ranging instruments and techniques for measuring a distance between an ophthalmic ranging instrument and an eye of a subject.

### [Background]

Ophthalmic devices employ various techniques to image different parts of an eye of a subject and assess the eye's response to stimuli, for example, and are used by clinicians to diagnose and manage a variety of eye conditions. Ophthalmic devices include scanning laser ophthalmoscopes (SLOs), optical coherence tomography (OCT) imaging devices, fundus cameras, visual field-testing devices, microperimetry devices, and corneal topography devices (among others), or a combination of two or more such devices.

To acquire high quality images of the part of the eye that is of interest, ophthalmic imaging devices (such as SLOs and OCT scanners, for example) often require the position of the exit pupil of the ophthalmic imaging device to fall within a predetermined range of distances from or within the eye that is suitable for acquiring such images. For example, some wide-field retinal imaging devices require their exit pupil position to be brought into alignment with the centre of the pupil of the eye. Stereo range-finding devices (also referred to as stereo distance-measuring devices or stereo distance meters) are a kind of ophthalmic ranging instrument often used to measure distance to the eye, using stereoscopic ranging techniques. For example, some ophthalmic imaging devices include a so-called pupil alignment module (PAM), which is configured to acquire stereo images of the eye, locate respective pupil centres in the stereo images, and determine the distance between the PAM and the pupil based on a separation between the located pupil centres in the stereo images.

Known stereo range-finding devices are generally able to accurately measure their distance from the eye, provided that the eye is in focus in the acquired stereo images. However, as the stereo images become increasingly de-focused (i.e. blurred), the accuracy with which the distance can be measured decreases, and distance measurement can ultimately become impossible. The stereo cameras in stereo range-finding devices often employ inexpensive complementary metal oxide semiconductor (CMOS) sensors, and fast lenses of fixed focal length are typically used to compensate for the sensors' relatively low sensitivity to infra-red (IR) illumination that is typically employed for patient comfort and to avoid pupil constriction. As such lenses usually have a narrow depth-of-field, a conventional stereo range-finding device tends to produce reliable distance measurements only when the eye is located within a correspondingly narrow range of distances from the device. As the exit pupil position is typically adjusted by moving the ophthalmic imaging device towards and away from the eye, it would be desirable to be able to reliably measure the distance to the eye over a wide range of eye positions along the axial direction, for improved patent alignment and thus improved image quality. Further, known stereo range-finding devices typically employ one or more IR light sources to illuminate the eye when acquiring the stereo images. These light sources tend to produce specular highlights in the stereo images, which may obscure the edge of the pupil in the acquired images, at least at some distances. The locations of the pupil centres in the acquired stereo images may be made more difficult to detect as a result, making distance measurement less reliable or in some cases impossible even when the eye is within the depth of field of the stereo cameras.

It would therefore be desirable to provide an ophthalmic ranging instrument which at least partially overcomes one or more of the shortcomings of known ophthalmic ranging instruments noted above.

### [Summary]

There is provided, in accordance with a first example aspect herein, an ophthalmic apparatus arranged to determine an indication of a distance between the ophthalmic apparatus and an eye of a subject. The ophthalmic apparatus comprises an illumination device operable to illuminate the eye, and an imaging device operable to acquire an image of the eye, wherein the illumination device and the imaging device are arranged such that the image of the eye comprises a specular highlight from the illumination device having a spatial distribution in the image which varies with the distance between the ophthalmic apparatus and the eye. The ophthalmic apparatus further comprises a processor arranged to: process the image to determine a value of a spatial distribution indicator which is indicative of a property of the spatial distribution; and use the determined value of the spatial distribution indicator and at least one mapping which maps values of the spatial distribution indicator to corresponding values indicative of the distance between the ophthalmic apparatus and the eye to determine the indication of the distance between the ophthalmic apparatus and the eye.

In an example embodiment, the illumination device is operable to illuminate the eye from at least two different locations such that the specular highlight in the image comprises a first portion and a second portion that are set apart from one another in the image, the illumination device and the imaging device are arranged such that a separation between the first portion of the specular highlight and the second portion of the specular highlight varies with the distance between the ophthalmic apparatus and the eye, and the value of the spatial distribution indicator is indicative of the separation between the first portion and the second portion in the image. The illumination device may be arranged to provide point illumination of the eye from the at least two different locations such that the first portion of the specular highlight is a first spot in the image, and the second portion of the specular highlight is a second spot in the image.

In another example embodiment, the illumination device and the imaging device are arranged such that the specular highlight has a size or shape which varies with the distance between the ophthalmic apparatus and the eye, and the processor is arranged to process the image to determine, as the value of the spatial distribution indicator, a value indicative of the size or shape of the specular highlight.

There is provided, in accordance with a second example aspect herein, a system comprising the ophthalmic apparatus of the first example aspect or any of the example embodiments set out above, and a movement mechanism operable to move the ophthalmic apparatus towards and away from the eye, such that the spatial distribution of the specular highlight in the image varies with the movement. In this system, the processor is further arranged to determine whether the distance indicated by the determined indication is within a predefined range of values and, where the distance indicated by the determined indication is outside the predefined range of values, generate a control signal for adjustment of the distance by the movement mechanism towards the predefined range of values.

In an example embodiment of the system of the second example aspect, the ophthalmic apparatus is a stereo imaging apparatus comprising a further imaging device which is operable to acquire a further image of the eye. In this example embodiment, where the distance indicated by the determined indication is within the predefined range of values, the processor is further arranged to: process the acquired images using a stereoscopic ranging technique to determine a second indication of the distance between the ophthalmic apparatus and the eye; and determine whether the distance indicated by the determined second indication is outside a second predefined range of values and, where the distance indicated by the second indication is outside the second predefined range of values, generate a second control signal for adjustment of the distance between the ophthalmic apparatus and the eye by the movement mechanism towards the second predefined range of values. Furthermore, the processor may be arranged to determine the second indication of the distance between the ophthalmic apparatus and the eye by processing each image of the acquired images to locate a respective pupil centre of a pupil in the image, mapping the located pupil centres to a common image frame, determining a separation between the pupil centres in the common image frame, and determining the second indication of the distance based on the determined separation between the pupil centres.

The system of the second example aspect or any of the example embodiments thereof set out above may further comprise an ophthalmic imaging device for imaging the eye via an exit pupil position of the ophthalmic imaging device. In this case, the movement mechanism may be arranged to simultaneously move the exit pupil position and the ophthalmic apparatus towards and away from the eye, and the exit pupil position and the ophthalmic apparatus may be arranged relative to each other such that the exit pupil position of the ophthalmic imaging device is within a range of positions for imaging the eye when the distance between the ophthalmic apparatus and the eye is within the predefined range of values.

There is provided, in accordance with a third example aspect herein, a method of determining an indication of a distance between an ophthalmic apparatus and an eye of a subject. The method comprises: acquiring an image of the eye using illumination that produces a specular highlight in the image, the specular highlight having a spatial distribution in the image which varies with the distance between the ophthalmic apparatus and the eye; processing the image to determine a value of a spatial distribution indicator which is indicative of a property of the spatial distribution; and using the determined value of the spatial distribution indicator and at least one mapping which maps values of the spatial distribution indicator to corresponding values indicative of the distance between the ophthalmic apparatus and the eye to determine the indication of the distance between the ophthalmic apparatus and the eye.

In an example embodiment, the eye is illuminated from at least two different locations such that the specular highlight in the image comprises a first portion and a second portion that are set apart from one another in the image, a separation between the first portion of the specular highlight and the second portion of the specular highlight varies with the distance between the ophthalmic apparatus and the eye, and the value of the spatial distribution indicator is indicative of the separation between the first portion and the second portion in the image. In this example embodiment, the point illumination of the eye may be provided from the at least two different locations such that the first portion of the specular highlight is a first spot in the image, and the second portion of the specular highlight is a second spot in the image.

In another example embodiment, the specular highlight has a size or shape which varies with the distance between the ophthalmic apparatus and the eye, and the image is processed to determine, as the value of the spatial distribution indicator, a value indicative of the size or shape of the specular highlight.

The method of the third example aspect or any of its example embodiments set out above may further comprise determining whether the distance indicated by the determined indication is within a predefined range of values and, where the distance indicated by the determined indication is outside the predefined range of values, generating a control signal for adjustment of the distance between the ophthalmic apparatus and the eye towards the predefined range of values. This method may further comprise: acquiring a further image of the eye, such that the image and the further image are stereo images of the eye, and where the distance indicated by the determined indication is within the predefined range of values, processes of: processing the stereo images using a stereoscopic ranging technique to determine a second indication of the distance between the ophthalmic apparatus and the eye; and determining whether the distance indicated by the determined second indication is within a second predefined range of values. Where the distance indicated by the second indication is outside the second predefined range of values, the method may further comprise generating a second control signal for adjustment of the distance between the ophthalmic apparatus and the eye towards the second predefined range of values.

The second indication of the distance between the ophthalmic apparatus and the eye may be determined by processing each image of the acquired images to locate a respective pupil centre of a pupil in the image, mapping the located pupil centres to a common image frame, determining a separation between the pupil centres in the common image frame, and determining the second indication of the distance based on the determined separation between the pupil centres.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of a system for imaging an eye of a subject, which includes an ophthalmic apparatus according to an example embodiment herein.
Figure 2 is a schematic illustration showing details of the ophthalmic apparatus according to the example embodiment herein.
Figure 3 is a schematic illustration of an example implementation of the ophthalmic apparatus shown in Figure 2.
Figure 4A is a schematic side view of the ophthalmic apparatus shown in Figure 3, which shows one of the cameras and two of the LEDs of the ophthalmic apparatus.
Figure 4B is a schematic view along the z-axis of the ophthalmic apparatus shown in Figure 3, which shows the two cameras and the four LEDs of the ophthalmic apparatus.
Figure 5A to 5C show three example images acquired by an ophthalmic apparatus of an example embodiment at three different distances between the ophthalmic apparatus and the eye, where the ophthalmic apparatus is furthest from the eye in Figure 5A and closest to the eye in Figure 5C.
Figure 6 is a schematic illustration of a programmable signal processing hardware, which may be configured to perform the functions of the processor described herein.
Figure 7 is a flow diagram illustrating a process by which the processor of the example embodiment processes an image of an eye to determine a measure of the distance between the ophthalmic apparatus and the eye, and optionally uses the determined measure to generate a control signal for adjustment of the distance.
Figure 8 shows a representation in tabular form of a mapping used in example embodiments herein to determine an indication of the distance between the ophthalmic apparatus and the eye.
Figure 9 is a flow diagram illustrating a process by which the processor of the example embodiment may determine a second measure of the distance between the ophthalmic apparatus and the eye using a stereoscopic ranging technique, and use the determined second measure to generate a second control signal for adjustment of the distance.

### [Detailed Description of Example Embodiments]

In view of background provided above, the present inventors have devised distance-measuring techniques which use specular highlights (caused by reflections off the cornea of an eye, for example) to provide a measurement of a distance to the eye. The range-finding techniques may be implemented by an ophthalmic apparatus comprising an illumination device and an imaging device, wherein the illumination device and the imaging device are arranged relative to each other such that, in use of the ophthalmic apparatus, an image of the eye acquired by the imaging device comprises a specular highlight from the illumination device, where a spatial distribution in the image of the specular highlight varies as the distance between the ophthalmic apparatus and the eye varies. The ophthalmic apparatus further comprises a processor arranged to process the image to determine a value of a spatial distribution indicator which is indicative of a property of the spatial distribution (e.g. a size or shape of the specular highlight, or the separation between two separated portions thereof, for example), and use the determined value of the spatial distribution indicator to determine an indication (i.e. a measure) of the distance between the ophthalmic apparatus and the eye.

The range-finding techniques described herein have the advantage of not requiring the acquisition of a second (stereo or otherwise) image of the eye and the processing of the second image to determine the distance to the eye, at least for determining changes in the distance or obtaining estimates of the distance. The hardware and processing requirements are consequently less demanding. Furthermore, the range-finding techniques described herein may be more reliable than the known stereoscopic ranging (or range-finding) techniques described above when the acquired images are defocussed, as the specular highlight is typically a higher-contrast feature than the edge of the pupil, which is often difficult to distinguish from the iris in a defocussed (blurred) image. This is particularly the case where the illumination device produces a specular highlight in the images comprising two or more spots whose separation is used to quantify the distance to the eye, as the spots are easy to locate owing to their high contrast, and the locations of their centres are largely independent of the degree of blurring in the image.

The range-finding techniques described herein may complement the use of conventional stereoscopic range-finding techniques, for example by allowing the distance between the ophthalmic apparatus and the eye to be reliably brought to within a predetermined range so that any subsequently acquired stereo images are sufficiently focused for conventional stereoscopic range-finding techniques to deliver reliable results. Thus, in some example embodiments, the above-described image quality concerns may be addressed (at least in part) by using the range-finding techniques described herein to provide a reliable first measurement of the distance which is resilient to blurring in the image, before the distance is adjusted based on the first measurement to allow a conventional stereoscopic range-finding technique to be subsequently used for fine adjustment of the exit pupil position (or another reference position) of the ophthalmic imaging device relative to the eye.

Further, the range-finding techniques described herein may require for their implementation nothing more than some of the hardware of a typical conventional stereoscopic range-finding device, such as the PAM mentioned above. The above-described advantages can therefore be achieved with no associated increase in hardware complexity. More particularly, the inventors have recognised that the specular highlights, which have tended to reduce the reliability of conventional stereoscopic range-finding devices for the reasons explained above, may form the basis of an alternative approach to range-finding that is not bound by the limitations of the conventional stereoscopic range-finding techniques.

Example embodiments will now be described in detail with reference to accompanying drawings.

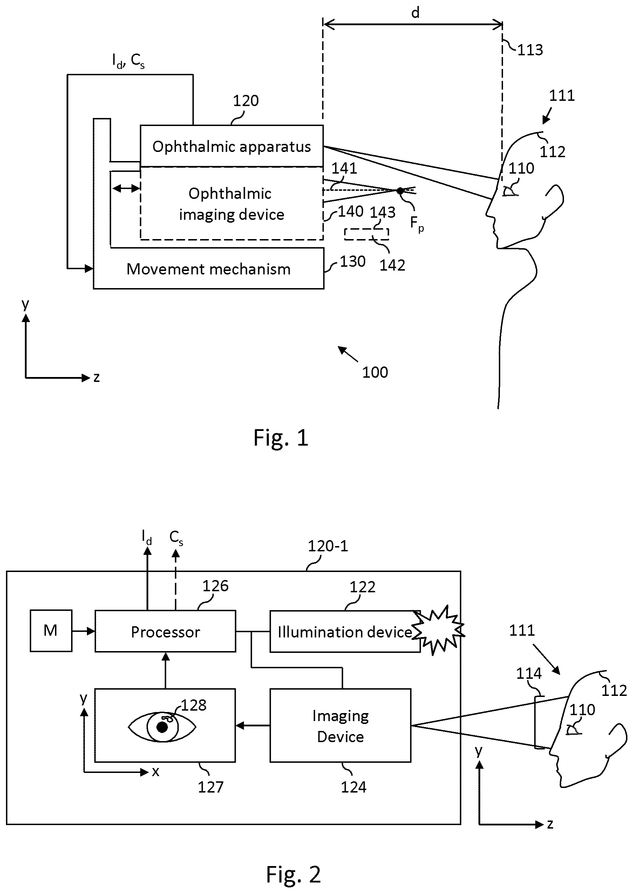

Figure 1 is a schematic illustration of a system 100 for imaging an eye 110 of a (human) subject 111. The system 100 comprises an ophthalmic apparatus 120 (which may also be referred to as an ophthalmic ranging instrument or ophthalmic ranging device), and a movement mechanism 130. The system 100 may, as in the present example embodiment, further comprise an ophthalmic imaging device 140.

The ophthalmic imaging device 140 is arranged to image the eye 110 via an exit pupil position F_{P} of the ophthalmic imaging device 140. The ophthalmic imaging device 140 may be operable to acquire an image of a retina of the eye 110 by illuminating a region of the retina via the exit pupil position Fₚ of the ophthalmic imaging device 140 and recording light reflected from the illuminated region and collected by the ophthalmic imaging device 140 via the exit pupil position Fₚ. The exit pupil position F_{P} is located along an imaging axis 141 of the ophthalmic imaging device 140, which extends along the same direction as the z-axis in Figure 1. The ophthalmic imaging device 140 may, as in the present example embodiment, be an optical coherence tomography (OCT) imaging device in the form of a swept-source OCT (SS-OCT) imaging device. The ophthalmic imaging device 140 may, however, be another kind of Fourier-domain OCT (FD-OCT) imaging device, such as a spectral-domain OCT (SD-OCT) imaging device or may alternatively be a time-domain OCT (TD-OCT) imaging device. In such cases, the OCT imaging device may be operable to acquire an image of a retina of the eye 110 by illuminating a region of the retina via the exit pupil position Fₚ and recording light reflected from the illuminated region and collected by the OCT imaging device via the exit pupil position Fₚ. However, the ophthalmic imaging device 110 is not so limited, and may be any other type of ophthalmic imaging device for imaging the eye 110 via an exit pupil position Fₚ of the ophthalmic imaging device 140, such as a scanning laser ophthalmoscope (SLO) or a fundus camera, for example.

The OCT imaging device may include well-known components, such as a light beam generator, a scanning system, an interferometer, a light detector and OCT data processing hardware (not shown). The scanning system may be arranged to perform a one- and/or two-dimensional point scan of a light beam across the retina of the eye 110 via an exit pupil position Fₚ of one or more lenses or a curved (e.g. ellipsoidal) mirror, and collect light which has been scattered by the retina via the exit pupil position Fₚ during the point scan. The OCT imaging device may thus acquire A-scans at respective scan locations that are distributed across a surface of the retina, by sequentially illuminating the scan locations with the light beam, one scan location at a time, and collecting at least some of the light scattered by the retina at each scan location. The OCT imaging device may be arranged to acquire OCT images in the form of B-scans by performing the point-scan to acquire successive A-scans along, for example, a straight line. However, the OCT imaging device may alternatively be arranged to acquire the B-scans by the scanning system performing line-scans, using hardware well-known to those versed in the art. More generally, the OCT imaging device may be arranged to acquire OCT images in the form of B-scans or C-scans by performing point-scans or a line-scans using predetermined scanning patterns well-known to those versed in the art (e.g. a spiral scan), or by employing a full-field set-up.

The system 100 may, as in the present example embodiment, further comprise a patient interface 142 having a contact surface 143. The contact surface 143 is arranged to contact the head 112 of the subject 111 during imaging of the eye 110 and thus fix the position of the eye 110 along the z-axis relative to the position of the contact surface 143. The patient interface 142 may, as in the present example embodiment, be provided in the form of a chinrest, which has an upward-facing contact surface 143 on which the chin of the subject 111 rests during imaging of the eye 110 by the ophthalmic imaging device 140. However, the contact surface 143 may be provided on another kind of patient interface, such as a mask contoured to fit around the eyes and over the nasal bridge of the subject 111 (referred to herein as "goggles"), through which the subject 111 looks during the imaging of the eye 110 by the ophthalmic imaging device 140, a forehead rest against which the forehead of the subject 111 presses during the imaging, a combination of the chinrest and the forehead rest, or one or two eyecups, for example.

The distance along the z-axis between the patient interface 142 and the ophthalmic imaging device 140 may, as in the present example embodiment, be adjusted by moving the ophthalmic imaging device 140 along the z-axis towards or away from the patient interface 142 while the patient interface 142 remains fixed in place (i.e. immobile in directions along the z-axis). For example, the patient interface 142 may be fixed to a table (not shown), and the ophthalmic imaging device 140 may be slidably mounted on the table so that it can be slid towards or away from the patient interface 142 along the z-axis, as required. However, the position of the patient interface 142 relative to the ophthalmic imaging device 140 may alternatively be adjustable by moving the patient interface 142 axially (i.e. along the z-axis) while the ophthalmic imaging device 140 remains fixed in place. For example, the ophthalmic imaging device 140 may be fixed to a table, and the patient interface 142 may be slidably mounted on the table so that can be slid relative to the ophthalmic imaging device 140. As a further alternative, the position of the patient interface 142 relative to the ophthalmic imaging device 140 may be adjustable by moving both the ophthalmic imaging device 140 and the patient interface 142 axially relative to the supporting table or other supporting structure. In all these cases, the position of the eye 110 relative to the ophthalmic imaging device 140 may be adjusted when preparing to image the eye 110 by adjusting the distance between the patient interface 142 and the ophthalmic imaging device 140.

The movement mechanism 130 may, as in the present example embodiment, be arranged to simultaneously move the exit pupil position F_{P} and the ophthalmic apparatus 120 towards or away from the eye 110, as required, thereby varying the spatial distribution of the specular highlight in the image. For example, the ophthalmic apparatus 120 may, as in the present example embodiment, be attached to the ophthalmic imaging device 140, and both may be moveable by the movement mechanism 130 forwards and backwards along the z-axis relative to the (fixed) patient interface 142. When preparing to image the eye 110, the movement mechanism 130 may, by moving the ophthalmic imaging device 140 axially (i.e. along the z-axis) relative to the patient interface 142, thus simultaneously move both the ophthalmic apparatus 120 and the exit pupil position F_{P} of the ophthalmic imaging device 140 in unison relative to the eye 110.

The movement mechanism 130 may, as in the present example embodiment, comprise a linear actuator (e.g. a rack and pinion) which is controllable by a processor (which may be the processor 126 described herein below) or by an operator, for example using buttons on a handset, to simultaneously move the exit pupil position F_{P} and the ophthalmic apparatus 120 towards or away from the eye 110, as described above. The exit pupil position F_{P} and the ophthalmic apparatus 120 are arranged relative to each other such that the exit pupil position F_{P} of the ophthalmic imaging device 140 is within a predetermined range of positions suitable for the imaging of a desired portion (e.g. a retina or an anterior segment) of the eye 110 when the distance between the ophthalmic apparatus 120 and the eye 110 is within a predefined range of values, as described below.

Although an adjustment of the distance between the patient interface 142 and the ophthalmic imaging device 140 as a whole is described above, the adjustment may instead be of a distance between the patient interface 142 and only some of the components of ophthalmic imaging device 140. For example, the axial position of the patient interface 142 may be fixed in relation to a portion of the ophthalmic imaging device 140 comprising (or consisting of) the interferometer, the detector, the light source, the OCT data processing hardware, and the remaining components of the ophthalmic imaging device 110, comprising (or consisting of) the scanning system, for example, may be moveable by the movement mechanism 130 relative to the aforementioned portion. Such an arrangement may be achieved by optically coupling the scanning system to the interferometer with an optical fibre, for example, and providing a translation mechanism employing a stepper motor, for example, to move the scanning system relative to the remaining components of the ophthalmic imaging device 140. The scanning system and the ophthalmic apparatus 120 may be provided in a fixed spatial arrangement which is moveable by the movement mechanism 130 along the z-axis such that the exit pupil position F_{P} of the ophthalmic imaging device 140 (which may be determined by optical elements in the scanning system) and the ophthalmic apparatus 120 are simultaneously moveable towards and away from the eye 110 so as to vary the spatial distribution of the specular highlight in the image.

In a further variant, the movement mechanism 130 is arranged to adjust the axial position of the exit pupil position F_{P} of the ophthalmic imaging device 140 by moving one or more optical elements of the scanning system, for example. In this variant, the axial position of the patient interface 142 relative to the ophthalmic imaging device 140 remains fixed, and the movement mechanism 130 is arranged to simultaneously move the exit pupil position F_{P} in unison with the ophthalmic apparatus 120 towards and away from the eye 110.

Further, although the system 100 described above comprises the patient interface 142, this is optional and may be omitted. For example, the patient may move to a predetermined position (e.g. sat upright in a chair in front of the ophthalmic imaging device 140), which is to be maintained during imaging, and the axial position of the ophthalmic imaging device 140 may then be adjusted as required. Therefore, where the ophthalmic apparatus 120 and exit pupil position Fₚ are at respective fixed axial positions relative to the ophthalmic imaging device 140, the movement mechanism 130 may simultaneously move the exit pupil position Fₚ and the ophthalmic apparatus 120 in unison towards or away from the eye 110, as described above. Similarly, the movement mechanism 130 may simultaneously move the exit pupil position Fₚ and the ophthalmic apparatus 120 relative to the ophthalmic imaging device 140 in cases where the position of the exit pupil position Fₚ of the ophthalmic imaging device 140 along the z-axis is adjustable relative to the ophthalmic imaging device 140, e.g. by moving or otherwise controlling one or more optical elements in the scanning system.

The ophthalmic apparatus 120 is arranged to determine at least one indication (or measure), I_{d}, of a distance, d, between the ophthalmic apparatus 120 and the eye 110 of the subject 111. The indication I_{d} may, as in the present example embodiment, be the value of the distance in the z-axis direction between the ophthalmic apparatus 120 and the eye 110, as shown with respect to a line 113 in a plane of the pupil of the eye 110 in Figure 1, which line is perpendicular to the z-axis direction. The indication of the distance may be provided in a variety of different forms, for example in terms of a distance between the ophthalmic apparatus 120 and the eye 110 expressed in metric or any other units of length (for example, as a multiple of a predetermined distance, e.g. 0.9D, 1.2D, etc.) or a distance between a suitable reference point and the eye 110 which is calculated using the distance d, for example.

Figure 2 is a schematic illustration showing details of an example implementation of the ophthalmic apparatus 120. The ophthalmic apparatus 120-1 of Figure 2 comprises an illumination device 122, an imaging device 124, and at least one processor 126.

The illumination device 122 is operable to illuminate the eye 110, preferably with infrared light, and the imaging device 124 is operable to acquire at least one image 127 of the eye 110. The image 127 may capture a portion 114 of the head 112 of the subject 111 comprising the whole of the eye 110, for example.

The illumination device 122 and the imaging device 124 are arranged relative to each other such that the image 127 of the eye 110 acquired by the imaging device 124 comprises a specular highlight 128 from the illumination device 122, the specular highlight 128 having a spatial distribution in the image 127 which varies with the distance d between the ophthalmic apparatus 120 and the eye 110. The specular highlight 128 may, as in the present example embodiment, be formed by the direct specular reflection of light from the illumination device 122 off the cornea of the eye 110, which acts as a convex reflector.

The variation of the spatial distribution of the specular highlight 128 with the distance d may be quantified by the variation with d of one or more measurable properties of the spatial distribution. For example, the property of the spatial distribution may, as in the present example embodiment, be a separation between spatially separated (i.e. unconnected) portions of the specular highlight 128, as described below. However, the property of the spatial distribution is not so limited and may alternatively be a size (e.g. length or area) or a shape of the specular highlight 128, for example a degree of curvature of an edge of the highlight or a degree of barrel distortion of the highlight. For example, the illumination device 122 may project a line segment of light onto the eye 110 (using a mask provided with a slit, for example), such that the specular highlight 128 takes the shape of a line segment in the image 127. In this case, the length of the linear specular highlight 128 may vary with the distance d, and may therefore be used to obtain a measure of the distance d. Additionally or alternatively, the linear specular highlight 128 may have a degree of curvature which varies with the distance d, and the degree of curvature may therefore be quantified and used to obtain a measure of the distance d.

Accordingly, one or more properties of the spatial distribution of the specular highlight 128 in the image 127 may be chosen, which contain information about the distance d between the ophthalmic apparatus 120 and the eye 110, and which may therefore be used by the processor 126 to determine the indication I_{d} of the distance d, as described in more detail below.

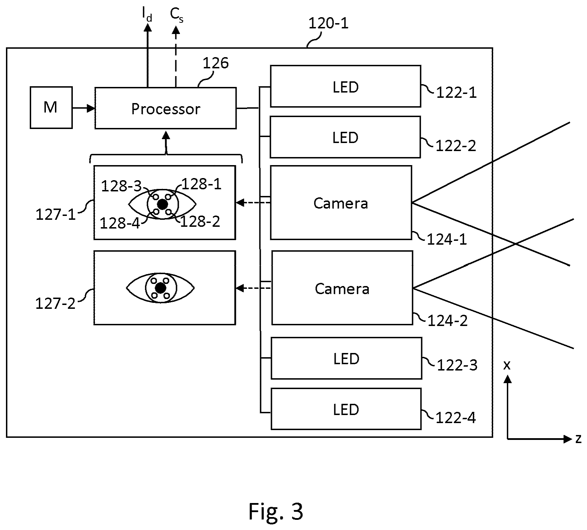

Figure 3 is a schematic illustration of an example implementation of the ophthalmic apparatus 120 of Figure 2. In the ophthalmic apparatus 120-1 illustrated in Figure 3, the imaging device 124 comprises a first camera 124-1 and a second camera 124-2, which are arranged to acquire stereo images 127-1 and 127-2 of the eye 110. The cameras 124-1 and 124-2 have respective CMOS sensors, although the form of the cameras is not so limited, and charge coupled device (CCD) sensors may be employed instead of CMOS sensors, for example. The first camera 124-1 and the second camera 124-2 are provided at the same position along the z-axis, and may have respective optical axes that are parallel to the imaging axis 141 of the ophthalmic imaging device 140. However the optical axes of the first camera 124-1 and the second camera 124-2 need not be parallel, and may converge towards the imaging axis 141, for example.

Furthermore, the illumination device 122 comprises four light-emitting diodes (LEDs) 122-1 to 122-4 in this example, which are preferably IR LEDs but may additionally or alternatively emit light at other (e.g. visible) wavelengths. These LEDs produce a respective specular highlight in each of the acquired images 127-1 and 127-2 which has four portions (in this case, spots) 128-1 to 128-4 that are set apart from one another, with their separation varying with the distance d.

It will be appreciated that this form of the illumination device 122 is only an example, and that fewer or more than four LEDs may instead be provided, in a variety of different arrangements such that the eye 110 can be illuminated from two or more different locations, and the specular highlight in the acquired images has portions that are set apart from one another, with their separation varying with the distance d. Furthermore, the illumination device 122 may illuminate the eye 110 from two or more different locations by means other than LEDs, for example from the ends of optical fibres arranged around the imaging device 124 or from apertures in a back-lit mask surrounding the imaging device 124 in the x-y plane.

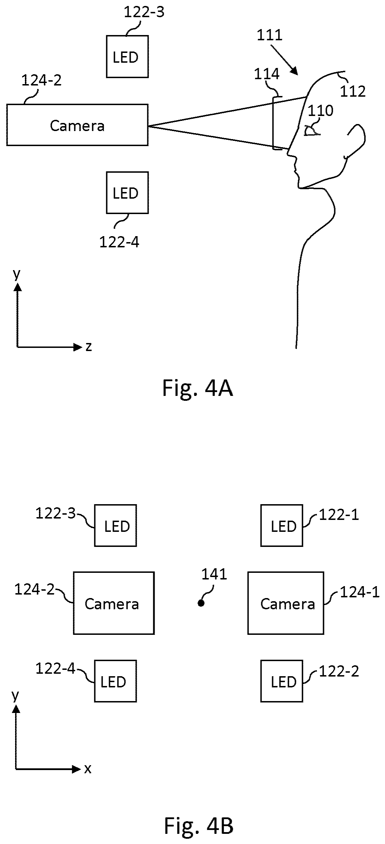

Figure 4A is a schematic side view of the cameras and LEDs of the ophthalmic apparatus 120-1 shown in Figure 3, while Figure 4B is a schematic view along the z-axis of the arrangement of cameras and LEDs of the ophthalmic apparatus 120-1 in the x-y plane. The illustrated positional relationship between the cameras 124-1 and 124-2, the LEDs 122-1 to 122-4, and the imaging axis 141 of the ophthalmic imaging apparatus 140, is fixed, and the cameras 124-1 and 124-2, and the LEDs 122-1 to 122-4, are arranged to move along the imaging axis in unison with the exit pupil position F_{P} of the ophthalmic imaging device 140, as described above.

When the ophthalmic apparatus 120-1 is implemented in the form of Figures 3, 4A, and 4B, use can be made of much of the hardware of a conventional stereo range-finding devices, although the processor 126 is arranged to process acquired images of the eye 110 using techniques described herein to measure distance d based on specular highlights in the images. In the example implementation of Figure 3, the ophthalmic apparatus 120-1 may also be used to provide an indication of the distance d between the ophthalmic apparatus 120-1 and the eye 110 using conventional stereoscopic ranging techniques, as described below.

The four LEDs 122-1 to 122-4 are arranged to provide point illumination of the eye 110 from their four different locations such that the specular highlight 128 in each of the images 127-1 and 127-2 of the eye 110 comprises four spots 128-1 to 128-4 that are set apart from one another, and are thus not connected to each other by another other portion of the specular highlight 128 in the image. Further, the LEDs 122-1 to 122-4 and the cameras 124-1 and 124-2 are arranged such that a respective separation between each pair of the four spots 128-1 to 128-4 in the image 127-1 and 127-2 varies with the distance d between the ophthalmic apparatus 120 and the eye 110. The separation between any two of the spots 128-1 to 128-4 may therefore be taken as a property of the spatial distribution of the specular highlight 127 which varies with distance d, as described herein.

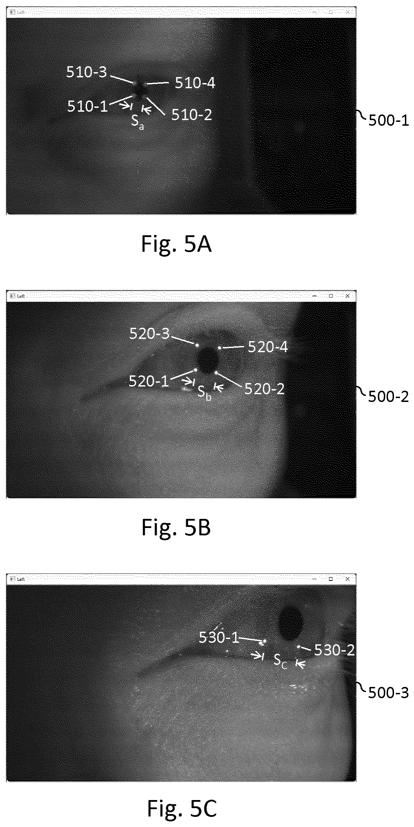

Figure 5A to 5C show three example images 500-1, 500-2 and 500-3, which were acquired by the first camera 124-1 at different respective distances dₐ, d_{b} and d_{c} , respectively, between the ophthalmic apparatus 120-1 and a left eye of a subject, where dₐ > d_{b} > d_{c}. Similar images of the left eye may be acquired by the second camera 124-2, from the different perspective of the second camera 124-2.

As shown in the first image 500-1, the arrangement of the four LEDs 122-1 to 122-4 relative to the first camera 124-1 result in a specular highlight which comprises four different spots 510-1 to 510-4, each corresponding to the direct specular reflection of light from a respective one of the LEDs 122-1 to 122-4 off the cornea of the eye. A separation, Sₐ, between the first spot 510-1 and the second spot 510-2 is present in the first image 500-1 taken when the distance between the ophthalmic apparatus 120-1 and the eye 110 was dₐ. In this example, the arrangement of the LEDs 122-1 to 122-4 is such that the four spots 510-1 to 510-4 form a square in the first image 500-1.

The second image 500-2 shown in Figure 5B was acquired when the distance between the ophthalmic apparatus 120-1 and the eye 110 was d_{b}, which is less than the distance dₐ. As shown in Figure 5B, the separation S_{b} between the first spot 520-1 (this being a less blurred and brighter version of the first spot 510-1 in Figure 5A) and the second spot 520-2 (this being a less blurred and brighter version of the second spot 510-2 in Figure 5A) has increased from Sₐ. The third image 500-3 shown in Figure 5C was acquired when the distance between the ophthalmic apparatus 120-1 and the eye 110 was d_{c}, which is less than the distance d_{b}. In Figure 5C, the separation S_{c} between the first spot 530-1 (corresponding to the first spot 520-1 in Figure 5B) and the second spot 530-2 (corresponding to the second spot 520-2 in Figure 5B) has increased from Sb.

Although the four LEDs 122-1 to 122-4 of the illumination device 122 provide point illumination of the eye 110 from their different respective locations, such that the specular highlight 128 in the image 127 of the eye 110 comprises four spots that are set apart from one another in the image 127, the illumination device 122 may more generally be arranged to illuminate the eye from at least two different locations, such that the specular highlight 128 in the acquired image 127 comprises a first portion and a second portion that are set apart from one another in the image 1127. For example, the illumination device 122 may comprise a single LED, which emits light through a mask comprising two apertures through which light from the LED may illuminate the eye 110. The first portion and the second portion in the resulting image may be spots or have any other desired shapes defined by the shapes of the corresponding apertures. In this more general case, the illumination device 122 and the imaging device 124 may be arranged such that a separation between the first portion of the specular highlight 128 and the second portion of the specular highlight 128 (as the measurable characteristic of the spatial distribution in this case) varies with the distance d between the ophthalmic apparatus 120 and the eye 110 in a similar manner is described with reference to Figures 5A to 5C.

Further, the second camera 124-2 is optional and need not be provided for the purpose of measuring the distance between the ophthalmic apparatus 120 and the eye 110, as the range-finding techniques described herein require no more than a single camera or other imaging device to capture the variation of the chosen property of the specular distribution of the specular highlight 128 with the distance between the ophthalmic apparatus 120 and the eye 110. The inclusion of the second camera 124-2 and the configuration of the processor 126 to process the image 127-2 acquired by the second camera 124-2 in the same way as image 127-1 may, however, improve the reliability of the ophthalmic apparatus 120-1 by allowing it to perform an additional distance measurement using a different approach that is not reliant on specular reflections, namely known stereoscopic ranging techniques. In addition, provision of the second camera 124-2 in addition to the first camera 124-1 may be allow changes in the captured images owing to movements of the eye 110 along the z-axis and x-axis to be disambiguated, allowing the position of the pupil along the x-axis to also be determined.

Although the second camera 124-2 is arranged to acquire at least one second image 127-2 of the eye 110 which comprises a specular highlight from the illumination device 122, the second camera 124-2 need not be arranged to capture any specular highlight from the illumination device 122, and may acquire a further image of the eye 110 without a specular highlight such that this image and the image acquired by the first camera 124-1 are stereo images that may be processed using known stereoscopic ranging techniques for distance measurement.

Referring again to Figure 2, the processor 126 is arranged to acquire the image 127 captured by the imaging device 124 using the illumination from the illumination device 122, as described above. The processor 126 may, as in the present example embodiment, control the illumination device 122 and the imaging device 124 to acquire the image 127 while the illumination device 122 illuminates the eye 110, and then receive the acquired image 127. The processor 126 is further arranged to process the image 127 using at least one mapping M to determine at least one indication I_{d} of the distance d between the ophthalmic apparatus 120 and the eye 110, as described below.

The processor 126 may, as in the present example embodiment, generate at least one control signal C_{S} for adjustment of the distance d between the ophthalmic apparatus 120 and the eye 110. For example, where the movement mechanism 130 is controllable by the processor 126, as in the present example embodiment, the control signal(s) C_{S} may control the movement mechanism 130 by direct control of the actuator(s) of the movement mechanism 130 or by instructing a controller of the movement mechanism 130. Alternatively, where the movement mechanism 130 is controllable by an operator, the control signal(s) C_{S} may instruct the operator (e.g. via a display of the system 100) to appropriately control the movement mechanism 130.

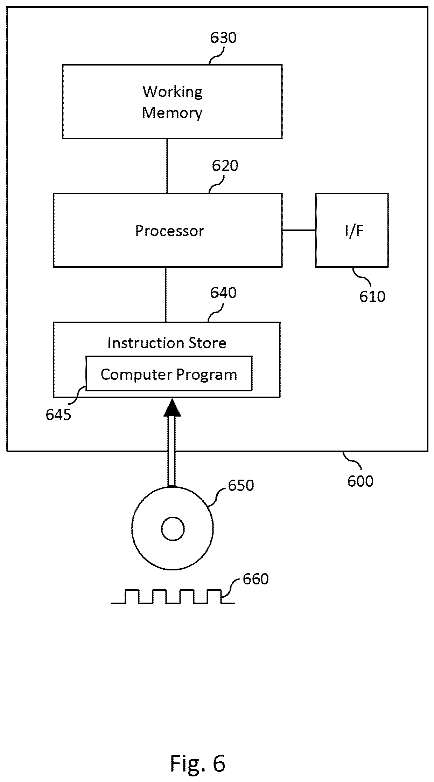

The processor 126 may be provided in any suitable form, for example as a processor 620 of a programmable signal processing hardware 600 of the kind illustrated schematically in Figure 6. The components of the programmable signal processing hardware 600 may be included within the ophthalmic apparatus 120. The programmable signal processing hardware 600 comprises a communication interface (I/F) 610, for receiving at least one image 127 from the imaging device 124. The I/F 610 may also receive one or more mappings M, and may output at least one indication I_{d} of the distance d between the ophthalmic apparatus 120 and the eye 110. The I/F 610 may output at least one control signal C_{S} for adjusting the distance d between the ophthalmic apparatus 120 and the eye 110 by the movement mechanism 130. The signal processing hardware 600 further comprises a processor 620 (e.g. a Central Processing Unit, CPU, and/or a Graphics Processing Unit, GPU), a working memory 630 (e.g. a random-access memory) and an instruction store 640 storing a computer program 645 comprising the computer-readable instructions which, when executed by the processor 620, cause the processor 620 to perform various functions of the processor 126 described herein. The working memory 630 stores information used by the processor 620 during execution of the computer program 645, including the mapping(s) M.

The instruction store 640 may comprise a ROM (e.g. in the form of an electrically erasable programmable read-only memory (EEPROM) or flash memory) which is pre-loaded with the computer-readable instructions. Alternatively, the instruction store 640 may comprise a RAM or similar type of memory, and the computer-readable instructions of the computer program 645 can be input thereto from a computer program product, such as a non-transitory, computer-readable storage medium 650 in the form of a CD-ROM, DVDROM, etc. or a computer-readable signal 660 carrying the computer-readable instructions. In any case, the computer program 645, when executed by the processor 620, causes the processor 620 to perform the functions of the processor 126 as described herein. In other words, the processor 126 of the present example embodiment may comprise a computer processor 620 and a memory 640 storing computer-readable instructions which, when executed by the computer processor 620, cause the computer processor 620 to perform the functions of the processor 126 as described herein.

It should be noted, however, that the processor 126 may alternatively be implemented in non-programmable hardware, such as an ASIC, an FPGA or other integrated circuit dedicated to performing the functions of the processor 126 described herein, or a combination of such non-programmable hardware and programmable hardware as described above with reference to Figure 6. Further, in some example embodiments, the programmable signal processing hardware 600 may further perform at least one of the functions of the OCT data processing hardware, where the ophthalmic imaging device 140 is an OCT imaging device, or functions of a controller of the movement mechanism 130 of the imaging device 124, where provided.

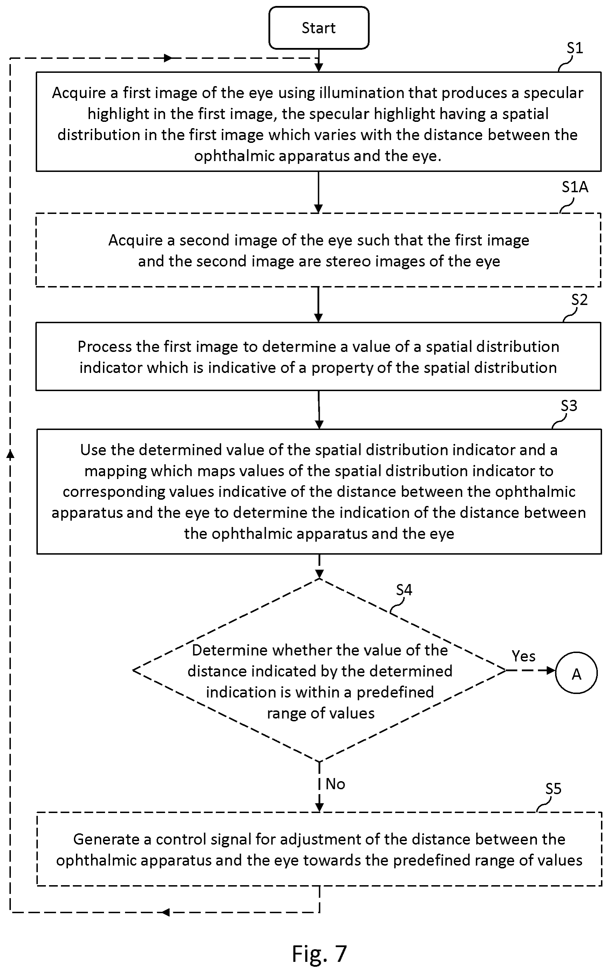

Figure 7 is a flow diagram illustrating a process by which the processor 126 processes the image 127 to determine at least one indication I_{d} of the distance d between the ophthalmic apparatus 120 and the eye 110.

In process S1 of Figure 7, the processor 126 acquires a first image 127-1 of the eye 110 using illumination which produces a specular highlight 128 in the first image 127-1. The processor 126 may, for example, receive the first image 127-1 from a memory (e.g. working memory 630), which stores the first image 127-1 after the first image 127-1 has been captured by the ophthalmic imaging device 140 under control of the processor 126 or otherwise. The processor 126 may acquire, as the first image 127-1, the image 500-1 of Figure 5A from the first camera 124-1, as described above.

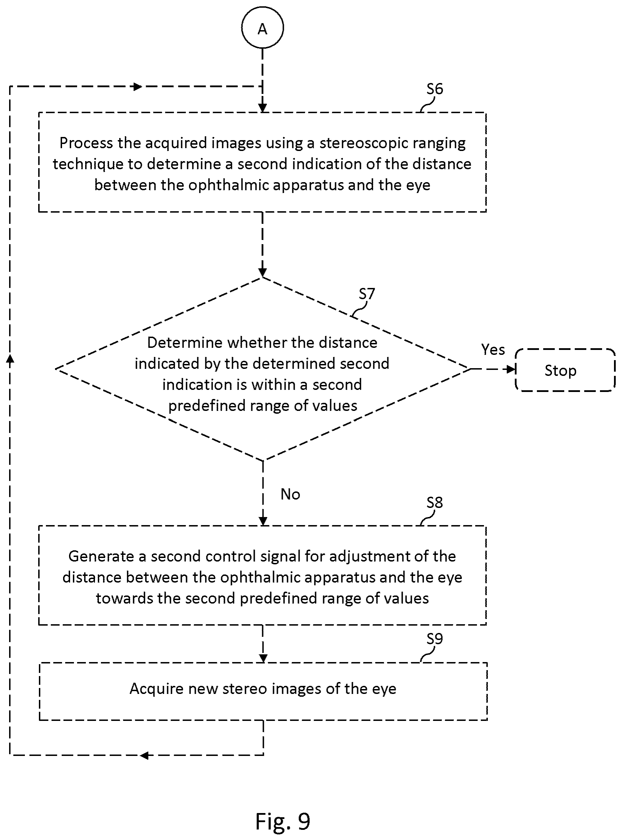

In optional process S1A of Figure 7, the processor 126 acquires a second image 127-2 of the eye 110, such that the first image 127-1 and the second image 127-2 are stereo images of the eye 110, as described above. This process is required for performing processes S6 to S8 of Figure 9 described below. The processor 126 may, for example, receive the second image 127-2 from a memory (e.g. working memory 630), which stores the second image 127-2 (as well as the first image 127-1) after the second image 127-2 has been captured by the ophthalmic imaging device 140 under control of the processor 126 or otherwise.

In process S2 of Figure 7, the processor 126 processes the first image 127-1 to determine a value of a spatial distribution indicator which is indicative of a property of the spatial distribution of the specular highlight 128. The spatial distribution indicator may quantify a property of the spatial distribution. The processor 126 may, as in the present example embodiment, identify a portion of the first image 127-1 corresponding to the specular reflection 128 in the first image 127-1 by using image segmentation techniques well-known to those skilled in the art, for example. The processor 126 may thus determine the spatial distribution using the identified portion of the first image 127-1.

As shown in Fig. 5A, the portions 510-1 to 510-4 of the first image 500-1 in the form of spots may be identified by the image segmentation. The centroids of the first spot 510-1 and the second spot 510-2 may then be calculated relative to the frame of the first image 500-1, and the distance between these centroids may be determined by the processor 126 as the value of the spatial distribution indicator. The determined distance is indicative of the separation Sₐ between the first spot 510-1 and the second spot 510-2.

Configuring the illumination device 122 to provide point illumination of the eye 110, such that the specular highlight 128 comprises spots, i.e. round or substantially round bright regions (as shown at 128-1 to 128-4 in Figure 3 and at 510-1 to 510-4 in Figure 5A, for example), may provide the advantage noted above of allowing the spots and their spacing to be detected reliably and accurately regardless of the degree of focus of the acquired image (i.e. the degree of blurring). This is because these round (or roundish) specular highlights stand out from surrounding regions in the image 127-1 of generally much lower brightness, and the highlights' respective centroid locations are largely unaffected by the degree of focus of the highlights in the image 127-1 and/or the sizes of the highlights.

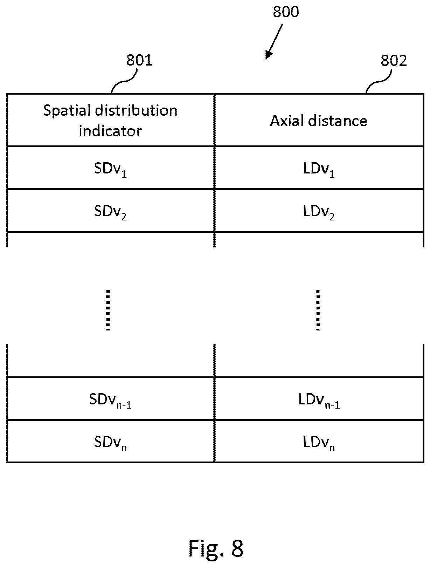

In process S3 of Figure 7, the processor 126 uses the determined value of the spatial distribution indicator and a mapping M, which maps values of the spatial distribution indicator to corresponding values indicative of the distance d between the ophthalmic apparatus 120 and the eye 110, to determine a first indication I_{d1} of the distance d between the ophthalmic apparatus 120 and the eye 110. Figure 8 shows a representation, in the form of a table 800, of the mapping M used in the present example embodiment to determine the first indication I_{d1} of the distance d. The table 800 comprises a first column 801 with values SDv₁, SDv₂,... SDvₙ of the spatial distribution indicator, and a second column 802 with corresponding values LDv₁, LDv₂,... LDvₙ of the distance d along the z-axis between the ophthalmic apparatus 120 and the eye 110. However, the form of the mapping M is not so limited, and it may alternatively be defined by a function, for example. The mapping M may be determined by calibration, specifically by acquiring a set of image of the eye 110 at different, measured distances from the ophthalmic apparatus 120, and determining how a measured separation between a pair of specular highlight spots varies with the distance.

In this way, the processor 126 determines the first indication I_{d1} of the distance d using the specular reflection 128 in the first image 127-1 acquired by the first camera 124-1. This range-finding technique may be used to quickly determine the distance d when bringing the subject 111 into a coarse alignment with the ophthalmic imaging device 140. Processes S4 and S5 of Figure 7 relate to an adjustment of the distance d between the ophthalmic apparatus 120 and the eye 110, which may be made using the first image 127-1 acquired by the first camera 124-1. These processes are optional and may be omitted where the above-described range-finding technique is used for other purposes (e.g. to merely provide information about the distance d).

In process S4 of Figure 7, the processor 126 determines whether the distance d indicated by the first indication I_{d1} is within a predefined first range of values, R₁. The first range R₁ may, for example, span values of the distance d at which images acquired by the imaging device 124 have a measured image quality, as measured by an acutance (i.e. sharpness) of the image, for example, which is above a predetermined threshold. The acutance may be quantified in various ways, for example by measuring image contrast, which may be done by calculating the variance (or standard deviation) of pixel values, for example. The acutance may alternatively be quantified using a calculated average gradient magnitude, or the amount of high-frequency spectral content in a fast Fourier transform (FFT) of the image, for example.

As an example, the range of values R₁ of the distance d may be set to correspond to the depth-of-field of the first camera 124-1, whose limits are defined using a maximum acceptable circle of confusion. The maximum acceptable circle of confusion may be set for the stereoscopic ranging technique described herein to be able to determine an indication of the distance d, based on stereo images 127-1 and 127-2, with degree of confidence which is above a predetermined confidence threshold. The range of values R₁ may alternatively be defined as a predetermined range of distances which is centred on an exit pupil position of the first camera 124-1 (e.g. within 1%, 5% or 10% of the distance to the exit pupil position). In some example embodiments, the bounds of the first predetermined range of values R₁ may be set to respectively result in a minimum and a maximum size of the eye 110 being acquired in the image 127-1.

For example, Figures 5A and 5B show images 500-1 and 500-2 acquired when the corresponding distances between the ophthalmic apparatus 120-1 and the eye 110 were outside the first predefined range of values R₁. The image 500-1 was acquired by the first camera 124-1 when it was too far from the eye 110 to acquire a focused image. In this case, the value of the distance d indicated by the first indication I_{d1} is outside the predefined first range of values R₁. The image 500-2 in Figure 5B was acquired by the first camera 124-1 when it was almost close enough to the eye 110 for the first camera 124-1 and the second camera 124-2 to acquire stereo images that are suitable for use in the stereoscopic ranging process described below with reference to Figure 9. In this case, the value of the distance d indicated by the first indication I_{d1} is within the predefined first range of values R₁. Figure 5C shows an image 500-3 acquired too close to the eye 110, such that the value of the distance d indicated by the first indication I_{d1} is not within the predefined first range of values R₁.

Where the value of the distance d indicated by the first indication I_{d1} has been determined in process S4 to not be within the predefined first range of values R₁ then, in process S5 of Figure 7, the processor 126 generates a first control signal Cₛ₁ for adjustment of the distance d between the ophthalmic apparatus 120 and the eye 110 towards the predefined first range of values R₁. The first control signal Cₛ₁ may, as in the present example embodiment, control the movement mechanism 130 to adjust the distance d towards the predefined first range of values R₁. However, the first control signal Cₛ₁ may alternatively instruct the operator to control the movement mechanism 130 to adjust the distance d towards the first range of values R₁, as described above.

In some example embodiments, the processor 126 repeats processes S1 to S5 by looping from process S5 back to process S1 of Figure 7, thus acquiring a new first image of the eye 110 in the repeat of process S1, and subsequently processing the new first image in repeats of processes S2 and S3, and repeating processes S4 and S5 based on a newly determined value of the distance, before again looping back to process S1, etc. This repeated performance of processes S1 to S5 continues until it is determined in a repeat of process S4 that the value of the distance d indicated by the first indication I_{d1} is within the predefined first range of values R₁. That is, the processor 126 acquires further images of the eye 110 captured by the first camera 124-1 and then processes these images, in turn, to determine a respective value of the first indication I_{d1} of the distance d, and make the determination of process S4 for each of the images. Where the value of the distance d indicated by the first indication I_{d1} has been determined in a repeat of process S4 to be within the predefined first range of values R₁, the process proceeds to S6 of Figure 9.

Figure 9 is a flow diagram illustrating further processes performed by the processor 126 when the value of the distance d indicated by the first indication I_{d1} is determined in S4 of Figure 7 to be within the predefined first range of values R₁. These further processes implement a stereoscopic range-finding technique, which may be performed after the range-finding technique based on analysis of the specular reflection 128 described above. However, these further processes are optional and may be omitted, in which case the ophthalmic imaging device 140 may begin imaging of the eye 110 in response to the processor 126 determining in process S4 of Figure 7 that the value of the distance indicated by the determined indication I_{d1} is within a predefined first range of values, R₁. In any case, the processor 126 may, after having determined that the value of the distance indicated by the determined indication I_{d1} is within a predefined first range of values, R₁. In process S4, generate an indicator indicating that no adjustment of the distance d between the ophthalmic apparatus 120 and the eye 110 based on determined first indication I_{d1} of the distance d is required.

In process S6 of Figure 9, the processor 126 may process both the first image 127-1 captured by the first camera 124-1 and the second image 127-2 captured by the second camera 124-2 using any known stereoscopic ranging technique, which uses the concept of parallax and triangulation to estimate distance, to determine a second indication I_{d2} of the distance d between the ophthalmic apparatus 120 and the eye 110. For example, the processor 126 may determine the second indication I_{d2} by processing each image of the acquired images to locate a respective pupil centre of a pupil in the image, mapping the located pupil centres to a common image frame, determining a separation between the pupil centres in the common image frame, and determining the second indication I_{d2} of the distance d using the determined separation, which is inversely related to the distance d.

In process S7 of Figure 9, the processor 126 determines whether the distance d indicated by the second indication I_{d2} is within a predefined second range of values R₂. The predefined second range of values R₂ may, as in the present example embodiment, be a range of values of the distance d for which the exit pupil position F_{P} of the ophthalmic imaging device 140 is within the predetermined portion of the eye 110 (along the z-axis) that is suitable for imaging the eye 110. For example, the range of values of the distance d may be such that the exit pupil position F_{P} of the ophthalmic imaging device 140 falls, in the axial direction, within a predetermined distance (e.g. within 0.01 mm, 0.1 mm or 1 mm) from the plane of the pupil of the eye 110.

In process S8 of Figure 9, where the determination in process S7 is that the distance d indicated by the second indication Iₛ₂ is outside the second predefined range of values R₂, the processor 126 generates a second control signal Cₛ₂ for adjustment of the distance d between the ophthalmic apparatus 120 and the eye 110 towards the second predefined range of values R₂. The second control signal Cₛ₂ may be used to adjust the distance d in the manner as described above for the first control signal Cₛ₁. Where the second predefined range of values R₂ is such that the exit pupil position F_{P} of the ophthalmic imaging device 140 is within the predetermined portion of the eye 110, the second control signal Cₛ₂ adjusts the distance d towards the second predefined range of values R₂ and, due to the configuration of the movement mechanism 130 to simultaneously move the exit pupil position F_{P} of the ophthalmic imaging device 140 and the ophthalmic apparatus 120 towards and away from the eye 110 (as described above), this results in the simultaneous adjustment of the exit pupil position F_{P} towards the predetermined portion of the eye 110.

In some example embodiments, the processor 126 repeats processes S6 to S8, as shown in Figure 9, until it is determined in a repeat of process S7 that the distance d indicated by a repeat determined second indication I_{d2} is within the second predefined range of values R₂. In this case, the processor 126 acquires further stereo images of the eye captured by the first camera 124-1 and the second camera 124-2 in process S9 of each iteration and then processes these newly acquired images in a repeat of process S6 to determine a respective second indication I_{d2} of the distance d between the ophthalmic apparatus 120 and the eye 110, before making the determination of process S7 using the latest second indication I_{d2} in each iteration.

Where the determination in process S7 of Figure 9 is that the distance d indicated by the determined second indication Iₛ₂ is within the second predefined range of values R₂, the ophthalmic imaging device 140 may, in some example embodiments, start imaging the eye 110.

Embodiments described above are summarised in the following numbered clauses E1 to E15.
E1. An ophthalmic apparatus (120) arranged to determine an indication of a distance (d) between the ophthalmic apparatus and an eye (110) of a subject, the ophthalmic apparatus (120) comprising:
   an illumination device (122) operable to illuminate the eye (110);
   an imaging device (124) operable to acquire an image (127; 127-1) of the eye (110);
   wherein the illumination device (122) and the imaging device (124) are arranged such that the image (127; 127-1) of the eye (110) comprises a specular highlight (128) from the illumination device (122) having a spatial distribution in the image (127; 127-1) which varies with the distance (d) between the ophthalmic apparatus (120) and the eye (110); and
   a processor (126) arranged to:
      process the image (127; 127-1) to determine a value of a spatial distribution indicator which is indicative of a property of the spatial distribution; and
      use the determined value of the spatial distribution indicator and at least one mapping (M) which maps values of the spatial distribution indicator to corresponding values indicative of the distance between the ophthalmic apparatus (120) and the eye (110) to determine the indication of the distance (d) between the ophthalmic apparatus (120) and the eye (110).
E2. The ophthalmic apparatus (120) of E1, wherein
   the illumination device (122) is operable to illuminate the eye (110) from at least two different locations such that the specular highlight (128) in the image comprises a first portion (128-1) and a second portion (128-2) that are set apart from one another in the image (127-1),
   the illumination device (122) and the imaging device (124) are arranged such that a separation between the first portion (128-1) of the specular highlight and the second portion (128-2) of the specular highlight varies with the distance (d) between the ophthalmic apparatus (120) and the eye (110), and
   the value of the spatial distribution indicator is indicative of the separation between the first portion (128-1) and the second portion (128-2) in the image (127-1).
E3. The ophthalmic apparatus (120) of E2, wherein the illumination device (122-1 to 122-4) is arranged to provide point illumination of the eye (110) from the at least two different locations such that the first portion (128-1) of the specular highlight is a first spot in the image (127-1), and the second portion (128-2) of the specular highlight is a second spot in the image (127-1).
E4. The ophthalmic apparatus (120) of E1, wherein
   the illumination device (122) and the imaging device (124) are arranged such that the specular highlight (128) has a size or shape which varies with the distance (d) between the ophthalmic apparatus (120) and the eye (110), and
   the processor (126) is arranged to process the image (127) to determine, as the value of the spatial distribution indicator, a value indicative of the size or shape of the specular highlight (128).
E5. A system (100) comprising:
   the ophthalmic apparatus (120) of any of E1 to E4; and
   a movement mechanism (130) operable to move the ophthalmic apparatus (120) towards and away from the eye (110), such that the spatial distribution of the specular highlight (128) in the image (127) varies with the movement,
   wherein the processor (126) is further arranged to determine whether the distance indicated by the determined indication is within a predefined range of values and, where the distance indicated by the determined indication is outside the predefined range of values, generate a control signal (Cₛ) for adjustment of the distance (d) by the movement mechanism (130) towards the predefined range of values.
E6. The system (100) of E5, wherein
   the ophthalmic apparatus (120) is a stereo imaging apparatus comprising a further imaging device (124-2) which is operable to acquire a further image (127-2) of the eye (110), and
   where the distance indicated by the determined indication is within the predefined range of values, the processor (126) is further arranged to:
      process the acquired images (127-1, 127-2) using a stereoscopic ranging technique to determine a second indication of the distance (d) between the ophthalmic apparatus (120) and the eye (110); and
      determine whether the distance indicated by the determined second indication is within a second predefined range of values and, where the distance indicated by the second indication is outside the second predefined range of values, generate a second control signal for adjustment of the distance (d) between the ophthalmic apparatus (120) and the eye (110) by the movement mechanism (130) towards the second predefined range of values.
E7. The system (100) of E6, wherein the processor (126) is arranged determine the second indication of the distance between the ophthalmic apparatus (120) and the eye (110) by processing each image (127-1; 127-2) of the acquired images (127-1, 127-2) to locate a respective pupil centre of a pupil in the image, mapping the located pupil centres to a common image frame, determining a separation between the pupil centres in the common image frame, and determining the second indication of the distance based on the determined separation between the pupil centres.
E8. The system (100) of any one of E5 to E7, further comprising:
   an ophthalmic imaging device (140) for imaging the eye (110) via an exit pupil position (F_{P}) of the ophthalmic imaging device (140),
   wherein the movement mechanism (130) is arranged to simultaneously move the exit pupil position (F_{P}) and the ophthalmic apparatus (120) towards and away from the eye (110), and
   wherein the exit pupil position (F_{P}) and the ophthalmic apparatus (120) are arranged relative to each other such that the exit pupil position (F_{P}) of the ophthalmic imaging device (140) is within a range of positions for imaging the eye (110) when the distance between the ophthalmic apparatus (120) and the eye (110) is within the predefined range of values.
E9. A method of determining an indication of a distance between an ophthalmic apparatus (120) and an eye (110) of a subject, the method comprising:
   acquiring (S1) an image (127) of the eye (110) using illumination that produces a specular highlight (128) in the image (127), the specular highlight (128) having a spatial distribution in the image (127) which varies with the distance (d) between the ophthalmic apparatus (120) and the eye (110);
   processing (S2) the image (127) to determine a value of a spatial distribution indicator which is indicative of a property of the spatial distribution; and
   using (S3) the determined value of the spatial distribution indicator and at least one mapping (M) which maps values of the spatial distribution indicator to corresponding values indicative of the distance between the ophthalmic apparatus (120) and the eye (110) to determine the indication of the distance (d) between the ophthalmic apparatus (120) and the eye (110).
E10. The method of E9, wherein
   the eye (110) is illuminated from at least two different locations such that the specular highlight (128) in the image (127-1) comprises a first portion (128-1) and a second portion (128-2) that are set apart from one another in the image (127-1),
   a separation between the first portion (128-1) of the specular highlight (128) and the second portion (128-2) of the specular highlight (128) varies with the distance (d) between the ophthalmic apparatus (120) and the eye (110), and
   the value of the spatial distribution indicator is indicative of the separation between the first portion (128-1) and the second portion (128-2) in the image (127-1).
E11. The method of E10, wherein point illumination of the eye (110) is provided from the at least two different locations such that the first portion (128-1) of the specular highlight (128) is a first spot in the image (127-1), and the second portion (128-2) of the specular highlight (128) is a second spot in the image (127-1).
E12. The method of E9, wherein
   the specular highlight (128) has a size or shape which varies with the distance (d) between the ophthalmic apparatus (120) and the eye (110), and
   the image (127) is processed to determine, as the value of the spatial distribution indicator, a value indicative of the size or shape of the specular highlight (128).
E13. The method of any one of E9 to E12, further comprising determining whether the distance (d) indicated by the determined indication is within a predefined range of values and, where the distance (d) indicated by the determined indication is outside the predefined range of values, generating a control signal (Cₛ) for adjustment of the distance between the ophthalmic apparatus (120) and the eye (110) towards the predefined range of values.
E14. The method of E13, further comprising:
   acquiring (S1A) a further image (127-2) of the eye (110), such that the image (127-1) and the further image (127-2) are stereo images of the eye (110),
   where the distance indicated by the determined indication is within the predefined range of values, processes of:
      processing the stereo images (127-1, 127-2) using a stereoscopic ranging technique to determine a second indication of the distance between the ophthalmic apparatus (120) and the eye (110);
      determining whether the distance indicated by the determined second indication is within a second predefined range of values; and
      where the distance indicated by the second indication is outside the second predefined range of values, generating a second control signal for adjustment of the distance between the ophthalmic apparatus (120) and the eye (110) towards the second predefined range of values.
E15. The method of E14, wherein the second indication of the distance between the ophthalmic apparatus (120) and the eye (110) is determined by processing each image (127-1; 127-2) of the acquired images (127-1, 127-2)to locate a respective pupil centre of a pupil in the image (127-1; 127-2), mapping the located pupil centres to a common image frame, determining a separation between the pupil centres in the common image frame, and determining the second indication of the distance based on the determined separation between the pupil centres.

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the function of the processor 126, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the processor 126 may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

While this specification contains many specific embodiment details, these should not be construed as limitations on the scope of any inventions or of what may be claimed, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A system (100) comprising:
a stereo ophthalmic apparatus (120) arranged to determine an indication of a distance (d) between the stereo ophthalmic apparatus (120) and an eye (110) of a subject, the stereo ophthalmic apparatus (120) comprising:
an illumination device (122) operable to illuminate the eye (110);
an imaging device (124) operable to acquire an image (127; 127-1) of the eye (110);
a further imaging device (124-2) which is operable to acquire a further image (127-2) of the eye (110);
wherein the illumination device (122) and the imaging device (124) are arranged such that the image (127; 127-1) of the eye (110) comprises a specular highlight (128) from the illumination device (122) having a spatial distribution in the image (127; 127-1) which varies with the distance (d) between the stereo ophthalmic apparatus (120) and the eye (110); and
a processor (126) arranged to:
process the image (127; 127-1) to determine a value of a spatial distribution indicator which is indicative of a property of the spatial distribution; and
use the determined value of the spatial distribution indicator and at least one mapping (M) which maps values of the spatial distribution indicator to corresponding values indicative of the distance between the stereo ophthalmic apparatus (120) and the eye (110) to determine the indication of the distance (d) between the stereo ophthalmic apparatus (120) and the eye (110),
the system (100) further comprising a movement mechanism (130) operable to move the stereo ophthalmic apparatus (120) towards and away from the eye (110), such that the spatial distribution of the specular highlight (128) in the image (127) varies with the movement, wherein
the processor (126) is further arranged to determine whether the distance indicated by the determined indication is within a predefined range of values,
where the distance indicated by the determined indication is outside the predefined range of values, the processor (126) is further arranged to generate a control signal (Cₛ) for adjustment of the distance (d) by the movement mechanism (130) towards the predefined range of values, and
where the distance indicated by the determined indication is within the predefined range of values, the processor (126) is further arranged to:
process the acquired images (127-1, 127-2) using a stereoscopic ranging technique to determine a second indication of the distance (d) between the stereo ophthalmic apparatus (120) and the eye (110);
determine whether the distance indicated by the determined second indication is within a second predefined range of values; and
where the distance indicated by the second indication is outside the second predefined range of values, generate a second control signal for adjustment of the distance (d) between the stereo ophthalmic apparatus (120) and the eye (110) by the movement mechanism (130) towards the second predefined range of values.

2. The system (100) of Claim 1, wherein
the illumination device (122) is operable to illuminate the eye (110) from at least two different locations such that the specular highlight (128) in the image comprises a first portion (128-1) and a second portion (128-2) that are set apart from one another in the image (127-1),
the illumination device (122) and the imaging device (124) are arranged such that a separation between the first portion (128-1) of the specular highlight and the second portion (128-2) of the specular highlight varies with the distance (d) between the stereo ophthalmic apparatus (120) and the eye (110), and
the value of the spatial distribution indicator is indicative of the separation between the first portion (128-1) and the second portion (128-2) in the image (127-1).

3. The system (100) of Claim 2, wherein the illumination device (122-1 to 122-4) is arranged to provide point illumination of the eye (110) from the at least two different locations such that the first portion (128-1) of the specular highlight is a first spot in the image (127-1), and the second portion (128-2) of the specular highlight is a second spot in the image (127-1).

4. The system (100) of Claim 1, wherein
the illumination device (122) and the imaging device (124) are arranged such that the specular highlight (128) has a size or shape which varies with the distance (d) between the stereo ophthalmic apparatus (120) and the eye (110), and
the processor (126) is arranged to process the image (127) to determine, as the value of the spatial distribution indicator, a value indicative of the size or shape of the specular highlight (128).

5. The system (100) of any preceding claim, wherein the processor (126) is arranged determine the second indication of the distance between the stereo ophthalmic apparatus (120) and the eye (110) by processing each image (127-1; 127-2) of the acquired images (127-1, 127-2) to locate a respective pupil centre of a pupil in the image, mapping the located pupil centres to a common image frame, determining a separation between the pupil centres in the common image frame, and determining the second indication of the distance based on the determined separation between the pupil centres.

6. The system (100) of any preceding claim, further comprising:
an ophthalmic imaging device (140) for imaging the eye (110) via an exit pupil position (F_{P}) of the ophthalmic imaging device (140),
wherein the movement mechanism (130) is arranged to simultaneously move the exit pupil position (F_{P}) and the stereo ophthalmic apparatus (120) towards and away from the eye (110), and
wherein the exit pupil position (F_{P}) and the stereo ophthalmic apparatus (120) are arranged relative to each other such that the exit pupil position (F_{P}) of the ophthalmic imaging device (140) is within a range of positions for imaging the eye (110) when the distance between the stereo ophthalmic apparatus (120) and the eye (110) is within the predefined range of values.

7. A method of determining an indication of a distance between an ophthalmic apparatus (120) and an eye (110) of a subject, the method comprising:
acquiring (S1) an image (127) of the eye (110) using illumination that produces a specular highlight (128) in the image (127), the specular highlight (128) having a spatial distribution in the image (127) which varies with the distance (d) between the ophthalmic apparatus (120) and the eye (110);
acquiring (S1A) a further image (127-2) of the eye (110), such that the image (127-1) and the further image (127-2) are stereo images of the eye (110);
processing (S2) the image (127) to determine a value of a spatial distribution indicator which is indicative of a property of the spatial distribution;
using (S3) the determined value of the spatial distribution indicator and at least one mapping (M) which maps values of the spatial distribution indicator to corresponding values indicative of the distance between the ophthalmic apparatus (120) and the eye (110) to determine the indication of the distance (d) between the ophthalmic apparatus (120) and the eye (110);
determining whether the distance (d) indicated by the determined indication is within a predefined range of values;
where the distance (d) indicated by the determined indication is outside the predefined range of values, generating a control signal (Cₛ) for adjustment of the distance between the ophthalmic apparatus (120) and the eye (110) towards the predefined range of values; and
where the distance (d) indicated by the determined indication is within the predefined range of values, performing processes of:
processing the stereo images (127-1, 127-2) using a stereoscopic ranging technique to determine a second indication of the distance between the ophthalmic apparatus (120) and the eye (110);
determining whether the distance indicated by the determined second indication is within a second predefined range of values; and
where the distance indicated by the second indication is outside the second predefined range of values, generating a second control signal for adjustment of the distance between the ophthalmic apparatus (120) and the eye (110) towards the second predefined range of values.

8. The method of Claim 7, wherein
the eye (110) is illuminated from at least two different locations such that the specular highlight (128) in the image (127-1) comprises a first portion (128-1) and a second portion (128-2) that are set apart from one another in the image (127-1),
a separation between the first portion (128-1) of the specular highlight (128) and the second portion (128-2) of the specular highlight (128) varies with the distance (d) between the ophthalmic apparatus (120) and the eye (110), and
the value of the spatial distribution indicator is indicative of the separation between the first portion (128-1) and the second portion (128-2) in the image (127-1).

9. The method of Claim 8, wherein point illumination of the eye (110) is provided from the at least two different locations such that the first portion (128-1) of the specular highlight (128) is a first spot in the image (127-1), and the second portion (128-2) of the specular highlight (128) is a second spot in the image (127-1).

10. The method of Claim 7, wherein
the specular highlight (128) has a size or shape which varies with the distance (d) between the ophthalmic apparatus (120) and the eye (110), and
the image (127) is processed to determine, as the value of the spatial distribution indicator, a value indicative of the size or shape of the specular highlight (128).

11. The method of any one of Claims 7 to 10, wherein the second indication of the distance between the ophthalmic apparatus (120) and the eye (110) is determined by processing each image (127-1; 127-2) of the acquired images (127-1, 127-2)to locate a respective pupil centre of a pupil in the image (127-1; 127-2), mapping the located pupil centres to a common image frame, determining a separation between the pupil centres in the common image frame, and determining the second indication of the distance based on the determined separation between the pupil centres.
